# EUROPEAN PATENT SPECIFICATION

(11) **EP 3 068 363 B1**
(45) Date of publication and mention of the grant of the patent: **05.08.2020**
(21) Application number: 14796107.2
(22) Date of filing: 10.11.2014
(51) Int. Cl.: A61K 6/00

(54) **DENTAL COMPOSITION**
DENTALE ZUSAMMENSETZUNG
COMPOSITION DENTAIRE

(30) Priority: 11.11.2013 EP 13005318
(43) Date of publication of application: 21.09.2016
(73) Proprietor: Dentsply DeTrey GmbH, 78467 Konstanz (DE)
(72) Inventor: KLEE, Joachim E., 78315 Radolfzell (DE); MAIER, Maximilian, 40213 Düsseldorf (DE); LALEVÉE, Jacques, F-68200 Mulhouse (FR); FOUASSIER, Jean Pierre, F-68590 St. Hippolyte (FR); MORLET-SAVARY, Fabrice, F-68120 Pfastatt (FR)
(74) Representative: Dietz, Mirko
(86) International application number: PCT/EP2014/074209
(87) International publication number: WO 2015/067815

(56) References cited:
- MOSZNER N ET AL: "Benzoyl germanium derivatives as novel visible light photoinitiators for dental materials", DENTAL MATERIALS, ELSEVIER, AMSTERDAM, NL, vol. 24, no. 7, 1 July 2008 (2008-07-01), pages 901-907, XP022671145, ISSN: 0109-5641, DOI: 10.1016/J.DENTAL.2007.11.004 [retrieved on 2007-12-21]
- M.-A. TEHFE ET AL.: MACROMOL. RAPID COMMUN., vol. 31, 2010, pages 473-478, XP002722638, cited in the application
- BEATE GANSTER ET AL: "New Photocleavable Structures, 4", MACROMOLECULAR RAPID COMMUNICATIONS, vol. 29, no. 1, 2 January 2008 (2008-01-02), pages 57-62, XP055111343, ISSN: 1022-1336, DOI: 10.1002/marc.200700620

## Description

### Field of the invention

The present invention relates to a dental composition containing a specific photoinitiator compound which is useful for initiating radical polymerization of radical-polymerizable monomers contained in a photocurable dental composition. The present invention also relates to the use of the photoinitiator for the preparation of a photocurable dental composition.

A dental composition according to the present invention has increased polymerization efficiency and curing speed so that the mechanical properties of a cured dental composition may be improved.

### Background of the invention

The restoration of teeth commonly involves a light curable dental composition containing free-radically polymerizable resins. Light curing of a dental composition involves a photoinitiator system generating free radicals upon exposure to visible light. Free radicals may be typically produced by either of two pathways:
(1) the photoinitiator compound undergoes excitation by energy absorption with subsequent decomposition of the compound into one or more radicals (Norrish type I), or
(2) the photoinitiator compound undergoes excitation and the excited photoinitiator compound interacts with a second compound by either energy transfer or a redox reaction to form free radicals from any of the compounds (Norrish type II).

In order for a photoinitiator to be useful for use in a dental composition, the quantum yields indicating the conversion of light radiation to radical formation needs to be high since absorption or shielding of light by further components of the dental composition limit the amount of energy available for absorption by the photoinitiators. Accordingly, only 70 percent conversion of the polymerizable groups may be expected in a polymerization of a typical dental composition, whereby the mechanical strength of the polymerized dental composition is less than optimal and unreacted monomers may leach out from the polymerized dental composition. The leaching monomers may have detrimental effects. In order to alleviate this problem, multifuctional monomers are frequently used which are more likely to be included in the polymer network.

In addition, photoinitiators are required to have a high solubility, thermal stability, and storage stability when incorporated into a dental composition.

Finally, given that dental compositions usually contain (meth)acrylate or (meth)acrylamide monomers, free radical photocuring may be inhibited by the presence of oxygen. Oxygen inhibition is due to the rapid reaction of propagating radicals with oxygen molecules to yield peroxyl radicals which are not as reactive towards carbon-carbon unsaturated double bonds and therefore do not initiate or participate in any photopolymerization reaction. Oxygen inhibition may lead to premature chain termination and, therefore, incomplete photocuring.

Accordingly, the polymerization initiator system has a critical influence on the quality of the dental material. Camphor quinone in combination with tertiary amine is frequently used as photoinitiator system. However, the presence of amines in acrylate-containing compositions can cause yellowing in the resulting photocured composition, create undesirable odors, and soften the cured composition because of chain transfer reactions and therefore, often require the use of stabilizers. Moreover, the use of aromatic amines such as ethyl 4-dimethylamino benzoate gives rise to toxicological concerns.

Furthermore, it is desirable that the light activating the photoinitiator system has a long wavelength in order to avoid damage of soft tissue during polymerization of the dental composition in the patient's mouth. Accordingly, the photoinitiator system is required to contain a chromophoric group efficiently absorbing light of the desired wavelength in a range of from 400 to 800 nm. However, an increase of the absorption coefficient of the photoinitiator system increases the coloration of the photoinitiator system and thereby the coloration of the dental composition.

Accordingly, it is necessary that the chromophoric groups are efficiently destroyed during polymerization so that the coloration of the initiator system disappears in the polymerized dental composition. A destruction of the chromophoric groups during polymerization may also be useful in increasing the depth of cure of the dental composition since activating light is not shielded from unpolymerized layers of the dental composition by the photoinitiator system present in polymerized layers covering the unpolymerized layers.

N. Mozner et al., Dental Materials 24 (2008) 901-907 and Ganster, B. *et al.*, disclose benzoyl germanium derivatives as novel visible light photoinitiators for dental materials. Benzoyltrimethyl germanium is suggested to be cleaved by the irradiation of visible light. The primary alpha-cleavage products are suggested to be a benzoyl radical and a germyl radical. There is no evidence for the role of the germyl radical as a polymerization initiator. Rather, the benzoyl radical might be responsible for the observed activity. Accordingly, the activity of a germyl radical in a polymerisation reaction in a dental composition remains unknown.

M.-A. Tehfe et al. Macromol. Rapid Commun. 2010, 31, 473-478 disclose bis(germylketones) as type I photoinitiator systems sensitive above 500 nm. However, a dental composition or any composition comprising a particulate filler and/or solvent is not disclosed.

Beate Ganster et al.: "New Photocleavable Structures, 4", Macromol. Rapid Commun., vol. 29, no. 1, 2008, pages 57-62 discloses acylgermane-based photoinitiators for visible light curing. However, said photoinitiators are not used in combination with an iodonium salt as additional initiator.

### Summary of the invention

It is the problem of the present invention to provide a dental composition containing radical-polymerizable monomers which has an improved polymerization efficiency, high curing speed and high storage stability, and which does not give rise to coloration problems so that the mechanical strength of a dental composition may be improved as well as the adhesion of the polymerized dental composition to enamel and dentin. Moreover, it is the problem of the present invention to provide a use of a specific compound for the preparation of a dental composition.

The present invention provides a dental composition comprising
(a) one or more radical-polymerizable monomers,
(b) 0.1 to 7.0 percent by weight based on the total weight of radical polymerizable monomers in the composition of a compound of the following formula (I):

   R¹R²R³X(CO)X'R⁴R⁵R⁶ (I)

   wherein
   X and X' which may be the same or different, independently represent Si or Ge,
   R¹, R², R³, R⁴, R⁵, and R⁶, which may be the same or different, independently represent a hydrocarbon group having 1 to 20 carbon atoms, whereby any two hydrocarbon groups of R¹, R², R³, R⁴, R⁵, and R⁶ may be bonded to each other and form together with either X, X' or the moiety X(CO)X' to which they are attached a 3 to 12- membered heterocyclic ring,
   whereby any of the hydrocarbon group or the heterocyclic ring may
      - contain one or more keto groups other than the keto group of the X(CO)X' moiety, or
      - be substituted by one or more groups selected from halogen atoms, a cyano group, an amino group or a hydroxy group, and
      - in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group, and
(c) a particulate filler and/or solvent,
which contains an iodonium salt as an additional initiator.

Moreover, the present invention provides the use of a compound of the following formula (I):

R¹R²R³Ge(CO)GeR⁴R⁵R⁶ (I)

wherein
R¹, R², R³, R⁴, R⁵, and R⁶, which may be the same or different, independently represent a hydrocarbon group having 1 to 20 carbon atoms, whereby any two hydrocarbon groups of R¹, R², R³, R⁴, R⁵, and R⁶ may be bonded to each other and form together with either Ge or with the moiety Ge(CO)Ge to which they are attached a 3 to 12- membered heterocyclic ring,
whereby the hydrocarbon group or the heterocyclic ring may
   - contain one or more keto groups other than the keto group of the moiety Ge(CO)Ge, or
   - be substituted by one or more groups selected from halogen atoms, a cyano group, an amino group or a hydroxy group, and
   - in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group,
for the preparation of such a photocurable dental composition.

The present invention is based on the recognition that a ketone compound of formula (I) according to the present invention provides improved polymerization efficiency, high curing speed and high storage stability, and does not give rise to coloration problems of a dental composition comprising radical-polymerizable monomers. Accordingly, according to the present invention a relatively large amount of the dental composition can be photocured with reduced exposure to radiation. Due to the high efficiency of the photoinitiator compound, the presence of oxygen, or oxygen inhibition, is not a serious detriment during photocuring of a dental composition according to the present invention. Finally, since the photocuring speeds are high in a dental composition of the present invention, the dental compositions may be pigmented or filled so that light penetration is limited.

### Brief description of the Figures

Figure 1 shows photopolymerization profiles of HDDA in laminate: (1) without photoinitiator and (2) in the presence of DGK 1.5 % w/w; upon a Xenon lamp exposure filtered for λ > 400 nm.
Figure 2 shows photopolymerization profiles of TMPTA in laminate: (1) in the presence of DGK (2 % w/w) and (2) in the presence of DGK/diphenyliodonium hexafluorophosphate (2%/2% w/w); upon a laser diode irradiation@ 473 nm (100 mW/cm²).
Figure 3A shows the emission spectrum of the blue LED (SmartLite Focus - Dentsply). Figure 3B shows the emission spectrum of laser diodes (e.g. @532 nm).
Figure 4 shows the absorption spectrum of BSK in tert-butylbenzene and photolysis of BSK upon a SmartLite Focus exposure (40s, 80s, 120s, 160 s of irradiation).
Figure 5 shows ESR spectra obtained for the irradiation of a BSK solution (solvent = tert-butylbenzene): experimental (upper curve) and simulated (lower curve) spectra; SmartLite Focus exposure (20 s).
Figure 6 shows photolysis of a BSK/DPI solution (solvent = tert-butylbenzene/acetonitrile); [DPI] = 0.012 M upon a SmartLite Focus exposure (20s, 40s).
Figure 7 shows ESR spectra obtained for the irradiation of a BSK/DPI solution (solvent = tert-butylbenzene): experimental (upper curve) and simulated (lower curve) spectra; SmartLite Focus exposure (20 s).
Figure 8 shows bleaching of a BSK/DPI solution: (a) before and (b) after a SmartLite Focus exposure.
Figure 9 shows photopolymerization profiles of BisGMA/TEGDMA in laminate for different photoinitiating systems; irradiation upon blue LED (SmartLite Focus) or laser diode@532 nm.

### Detailed description of preferred embodiments

The term "polymerization" relates to the combining by covalent bonding of a large number of smaller molecules, such as monomers, to form larger molecules, that is, macromolecules or polymers. The monomers may be combined to form only linear macromolecules or they may be combined to form three-dimensional macromolecule, commonly referred to as crosslinked polymers. In case of a higher conversion rate of the polymerizable monomer, the amount of multifunctional monomers may be reduced or the leaching problem may be alleviated.

The terms "curing" and "photocuring" mean the polymerization of functional oligomers and monomers, or even polymers, into a crosslinked polymer network. Curing is the polymerization of unsaturated monomers or oligomers in the presence of crosslinking agents.

The terms "photocurable" and "curable" refer to a dental composition that will polymerize into a crosslinked polymer network when irradiated for example with actinic radiation such as ultraviolet (UV), visible, or infrared radiation.

The term " radical-polymerizable monomers" is meant to include any unsaturated material having one or more carbon-to-carbon double bonds (ethylenically unsaturated groups) capable of undergoing polymerization. The term encompasses unsaturated monomers, oligomers, and crosslinking agents. The singular form of the term is intended to include the plural. Oligomeric and multifunctional acrylates are examples of radical-polymerizable monomers.

The term "quantum yield" is used herein to indicate the efficiency of a photochemical process. More particularly, quantum yield is a measure of the probability that a particular molecule will absorb a quantum of light during its interaction with a photon. The term expresses the number of photochemical events per photon absorbed.

"Actinic radiation" is any electromagnetic radiation that is capable of producing photochemical action and can have a wavelength of at least 150 nm and up to and including 1250 nm, and typically at least 300 nm and up to and including 750 nm.

The present invention relates to a dental composition. The dental composition may be a dental adhesive composition, a dental composite, a resin modified dental cement, and a pit and fissure sealant. The dental composition may be cured by irradiation of actinic radiation.

The dental composition may be preferably an acidic dental composition having a pH in the range of from 0.5 to 6.5, more preferably 1 to 4.

The dental composition contains one or more radical-polymerizable monomers. The radical-polymerizable monomers may preferably be a polymerizable N-substituted alkyl acrylic or acrylic acid amide monomer or a (meth)acrylate compound.

A polymerizable N-substituted alkyl acrylic or acrylic acid amide monomer may be preferably selected from compounds characterized by one of the following formulas: 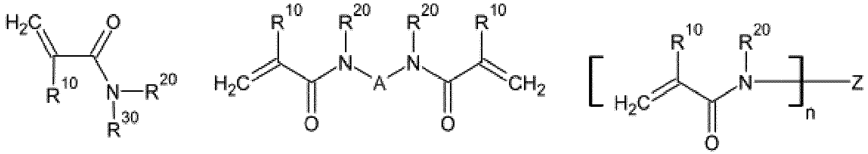 wherein R¹⁰, R²⁰ and R³⁰ independently represent a hydrogen atom or a C1 to C8 alkyl group; A represents a divalent substituted or unsubstituted organic residue having from 1 to 10 carbon atoms, whereby said organic residue may contain from 1 to 3 oxygen and/or nitrogen atoms; Z represents a saturated at least trivalent substituted or unsubstituted C1 to C8 hydrocarbon group, a saturated at least trivalent substituted or unsubstituted cyclic C3 to C8 hydrocarbon group, and n is at least 3. Preferably, radical-polymerizable monomers include bisacrylamides such as N,N'-diethyl-1,3-bisacrylamido-propan (BADEP), 1,3-bisacrylamido-propan (BAP), 1,3-bisacrylamido-2-ethyl-propan (BAPEN), N,N'-(2E)-but-2-en-1,4-diallylbis-[(N-prop-2-en-1) amid (BAABE) and N,N-di(cyclopropyl acrylamido) propane (BCPBAP).

A (meth)acrylate compound may be selected from the group of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Preferably, the polymerizable N-substituted alkyl acrylic or acrylic acid amide monomer or (meth)acrylate compound has a molecular weight of at most 400, more preferably at most 300.

Preferably, the radical-polymerizable monomers (a) each contain one or two radical-polymerizable groups.

It is preferable that a blending ratio of the radical-polymerizable monomers to the entire dental composition is 5 to 80% by weight. More preferably, the blending ratio is 10 to 60% by weight.

According to a preferred embodiment, the dental composition of the present invention comprises a radical-polymerizable monomer having an acidic group.

Moreover, the dental composition according to the present invention comprises 0.1 to 7.0 percent by weight based on the total weight of radical polymerizable monomers in the composition of a compound of the following formula (I):

R¹R²R³X(CO)X'R⁴R⁵R⁶ (I)

The compound of formula (I) may act as a type I photoinitiator whereby a single photon absorbed by the compound may generate two radicals R¹R²R³X and X'R⁴R⁵R⁶ which are able to polymerize the dental composition. Accordingly, the quantum yield of the compound formula (I) may be twice as high as an equimolar amount of camphor quinone/amine initiator.

Preferably, the dental composition contains less than 2.5 percent by weight, preferably 0.5 to 2.0 percent by weight based on the total weight of radical polymerizable monomers in the composition of a compound of formula (I).

In formula (I), X and X' which may be the same or different, independently represent Si or Ge.

According to a first embodiment, X and X' are the same. Accordingly, the compound of formula (I) is a bis(silyl) ketone of the following formula (II) or a bis(germyl) ketone of the following formula (III)

R¹R²R³Si(CO)SiR⁴R⁵R⁶ (II)

R¹R²R³Ge(CO)GeR⁴R⁵R⁶ (III)

According to a second embodiment, X is Si and X' is Ge according to the following formula (IV):

R¹R²R³Si(CO)GeR⁴R⁵R⁶ (IV)

In formula (I), (II), (III), and (IV), the moieties R¹, R², R³, R⁴, R⁵, and R⁶ may be the same or different, and independently represent a hydrocarbon group having 1 to 20 carbon atoms, preferably having 2 to 8 carbon atoms, and more preferably 2 to 6 carbon atoms.

The hydrocarbon group may be and alkyl group, a cycloalkyl group, a cycloalkylalkyl group, an aralkyl group or an aryl group.

An alkyl group may be straight-chain or branched C₁₋₂₀ alkyl group, typically a C₁₋₈ alkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a C₃₋₂₀ cycloalkyl group. Examples of the cycloalkyl group can include those having 3 to 14 carbon atoms, for example, cyclopropyl, cyclobutyl, cyclopentyl and cyclohexyl. A cycloalkylalkyl group can include those having 4 to 20 carbon atoms.

A cycloalkylalkyl group can include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl.

An aralkyl group may be a C₇₋₂₀ aralkyl group, typically a combination of a linear or branched alkyl group having 1 to 6 carbon atoms and an aryl group having 6 to 10 carbon atoms. Specific examples of an aralkyl group are a benzyl group or a phenylethyl group.

An aryl group can include aryl groups having 6 to 10 carbon atoms. Examples of the aryl group are phenyl and naphtyl. Aryl groups may contain 1 to 3 substituents. Examples of such substituents can include halogen atoms, a cyano group, a hydroxy group, an amino group, C₁₋₆ alkyl groups and C₁₋₆ alkoxy groups. Here, illustrative of the halogen atoms can be fluorine, chlorine, bromine and iodine. The C₁₋₄ alkyl groups are, for example, methyl, ethyl, n-propyl, isopropyl and n-butyl. Illustrative of the C₁₋₄ alkoxy groups are, for example, methoxy, ethoxy and propoxy. The alkyl moieties in these substituents may be linear, branched or cyclic.

Preferably, the hydrocarbon group is an aryl group selected from a phenyl group and a naphthyl group, which groups may optionally be substituted by one to three groups selected from halogen atoms, a cyano group, an amino group, a hydroxy group, C₁₋₆ alkyl groups and C1-6 alkoxy groups, or wherein the hydrocarbon group is a non-aromatic hydrocarbon group selected from a straight chain or branched alkyl group, a straight chain or branched alkenyl group, or a straight chain or branched alkynyl group.

The C₁₋₈ alkyl group and the C₃₋₁₄ cycloalkyl group may optionally be substituted by one or more members of the group selected from a C₁₋₄ alkyl group, C₁₋₄ alkoxy group, a phenyl group, and a hydroxy group. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

In formula (I), any two hydrocarbon groups of R¹, R², R³, R⁴, R⁵, and R⁶ may be bonded to each other and form together with either X, X' or the moiety X(CO)X' to which they are attached a 3 to 12- membered heterocyclic ring.

In formula (I), any of the hydrocarbon group or the heterocyclic ring may contain one or more keto groups other than the keto group of the X(CO)X' moiety,

Moreover, in formula (I), any of the hydrocarbon group or the heterocyclic ring may be substituted by one or more groups selected from halogen atoms, a cyano group, an amino group or a hydroxy group. Accordingly, in the hydrocarbon groups some or all hydrogen atoms are replaced by halogen atoms (e.g., fluoro, bromo, chloro), for example, halo-substituted alkyl groups such as chloromethyl, chloropropyl, bromoethyl and trifluoropropyl, and cyanoethyl.

In case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group. In case the hydrocarbon group is an alkyl group having more than one carbon atom, the alkyl group contains an alkylene Accordingly, in case the hydrocarbon group is an n-hexyl group, any of the carbon atoms of the alkylene chain excluding the terminal methyl group may be replaced by an oxygen atom, a sulfur atom, an amide group, an ester group, a urethane group or an NH group. Therefore, the following groups may be given as specific examples in case of one or more oxygen atoms:

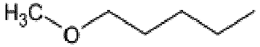

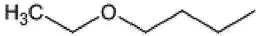

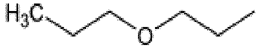

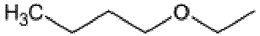

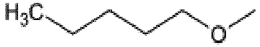

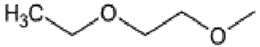

A preferable group of compounds are symmetrical compounds of the formula (I) wherein X and X' represents Ge, R¹ = R⁴, R² = R⁵, and R³ = R⁶, and R¹, R², R³, which may be the same or different, independently represent a C₁₋₆ alkyl group or a phenyl group.

A further preferable group of compounds are symmetrical compounds of the formula (I) wherein X and X' is Si, R¹ = R⁴, R² = R⁵, and R³ = R⁶, and R¹, R², R³, which may be the same or different, independently represent a C1-6 alkyl group or a phenyl group.

Specific examples for a compound of formula (I) include bis(triphenylgermyl)methanone, bis(triethylgermyl)methanone, and bis(trimethylgermyl)methanone.

A compound of the formula (I) may be a known compound which may be prepared according to published procedures. Alternatively, a compound of formula (I) may be prepared according to the following scheme (Johannesen R. et al, J. Chem. Soc. Perkin Trans. 1, 2000, 2677-2679).

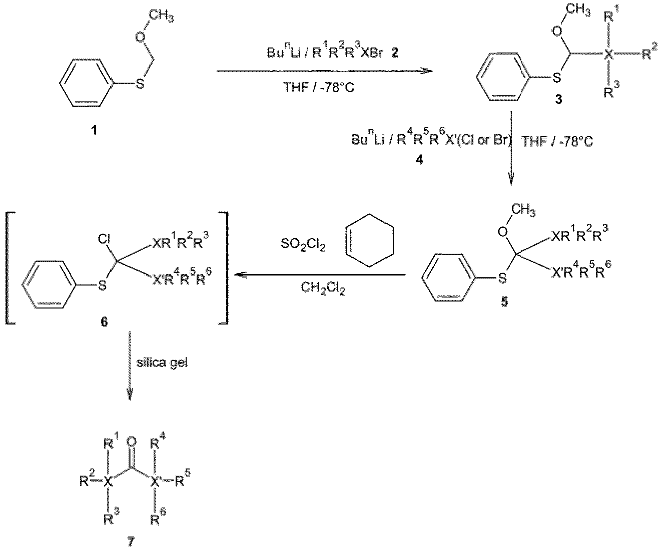

In the above scheme, X is preferably Ge.

According to the preparation method a compound **1** is treated with a strong base in an aprotic solvent at low temperature followed by reaction with a compound **2** for generating a compound **3.** The reaction temperature may be in the range of from - 20 to -100 °C, preferably -40 to -80 °C. The reaction time is not particularly limited and may be selected in the range of from 1 to 24 hours, preferably 2 to 10 hours. The reaction mixture may be quenched by the addition of water and the product may be extracted into benzene, washed with water, dried (MgSO₄) and evaporated to give **3.** The crude product may be used in the next step without further purification.

**3** may be treated with a strong base in an aprotic solvent at low temperature followed by reaction with a compound **4** for generating a compound **5.** The reaction temperature may be in the range of from 40 to -80 °C, preferably 30 to -20 °C. The reaction time is not particularly limited and may be selected in the range of from 1 to 24 hours, preferably 2 to 10 hours. The reaction mixture may be quenched by the addition an aqueous solution of NH₄Cl (10%) and the product may be extracted into benzene, washed with water, dried (MgSO₄) and evaporated to give **5.** The crude product may be used in the next step without further purification.

In case a symmetrical ketone is prepared, a compound **5** may be obtained in a single step by using two equivalents of **2.**

A compound **7** used in the present invention may be prepared by adding sulfuryl dichloride in dry dichloromethane dropwise to a solution of compound **5** in dry dichloromethane under nitrogen. The mixture is stirred for 30 min before a solution of cyclohexene in dry dichloromethane is added dropwise. The reaction temperature is not particularly limited and may be selected in the range of from 40 to -20 °C, preferably 0 °C. Subsequently, the solvent is removed and the residue is eluted through a silica gel column under inert atmosphere, preferably argon gas, using first degassed pentane as eluent, then a degassed mixture of pentane-diethyl ether 20:1 and a compound for formula (I) may be isolated as a compound of formula (I) of the present invention.

Alternatively, a symmetrical bis(germyl)ketone may also be prepared by adding to a trisubstituted germanium hydride such as triphenylgermanium hydride at low temperature such as at -23 °C, *n*-BuLi and subsequently dropwise ethyl formate (preferably freshly distilled over CaH₂) Subsequently, the reaction mixture may be allowed to warm at room temperature over before being quenched with aqueous hydrochloric acid. The organic phase is separated and the aqueous phase is extracted three times with diethyl ether. The combined organic phases are dried over sodium sulfate, filtered and concentrated to give the corresponding bis(germyl) methanol product. To a solution of bis(germyl) methanol in dry diethyl ether, successively dicyclohexylcarbodiimide and pyridinium trifluoroacetate were added. The flask is then carefully protected from light and dry DMSO is added dropwise. The stirring is continued overnight. The supernatant liquid may then then be withdrawn via syringe and filtered through a 0.2 µm syringe filter. After the filtrate is evaporated under vacuum in the absence of light, the desired bis(germyl)methanone is obtained.

Preferably, a compound of formula (I) has an absorption maximum in the range of from 400 nm to 900 nm. Accordingly, polymerization may be carried out by using blue or green LED light, preferably in the range of from 460 to 530 nm.

A tertiary amine may be used as a reducing material. Examples of the tertiary amine include triethanolamine, methyl 4-dimethylaminobenzoate, ethyl 4-dimethylaminobenzoate, dimethylaminoethyl methacrylate, or isoamyl 4-dimethylaminobenzoate.

In addition to these reducing materials, an organic metal compound, or a sulfinic acid derivative, can be used as a reducing material.

The additional initiator is an iodonium salt having the following formula: 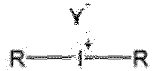 wherein the R which may be the same or different represent an aryl group which may be substituted, and Y⁻ is an anion selected from hexafluoroantimonate, trifluoromethylsulfate, hexafluorophosphate, tetrafluoroborate, hexafluoroarsenate, and tetraphenylborate.

Preferably, R is a phenyl group which may be substituted with 1 to 3 substituents selected from halogen atoms, a cyano group, a hydroxy group, an amino group, C₁₋₆ alkyl groups and C₁₋₆ alkoxy groups.

The additional initiator is used in a ratio of an amount of the compound of formula (I) to an amount of the additional initiator of 0.1 ≤ (mass of compound of formula (I)/additional initiator) ≤ 10.

The use of the additional initiator provides a synergistic effect in the case of iodonium salts. Preferred iodonium salts are diphenyliodonium hexafluorophosphate, and (4-methylphenyl) [4-(2-methylpropyl) phenyl] iodonium hexafluorophosphate (Irgacure 250, commercial product available from BASF SE).

The dental compositions of the present invention further contain particulate fillers and/ or solvents.

Suitable particulate fillers may be selected from fillers currently used in dental compositions. The filler should be finely divided and preferably has a maximum particle diameter less than about 100 µm and an average particle diameter less than about 10 µm. The filler may have a unimodal or polymodal (e.g., bimodal) particle size distribution.

The filler can be an inorganic material. It can also be a crosslinked organic material that is insoluble in the polymerizable resin, and is optionally filled with inorganic filler. The filler can be radiopaque, radiolucent or non-radiopaque. Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler may also be a filler obtainable by a process for the preparation of composite filler particles, comprising:
(a) coating a particulate filler having a median particle size (D50) of from 1 to 1200 nm with a coating composition containing a film-forming agent forming a coating layer on the surface of the particulate filler, said coating layer displaying reactive groups on the surface of the coating layer, said reactive groups being selected from addition polymerizable groups and step-growth polymerizable groups, thereby forming a coated particulate filler; subsequently or concurrently
(b) agglomerating the coated particulate filler, optionally in the presence of a further crosslinking agent and optionally in the presence of a further particulate filler not displaying reactive groups, for providing a granulation of the coated particulate filler wherein the granulation contains the coated particulate filler particles and the optional further particulate filler particles separated from and connected to each other by at least one coating layer, whereby the at least one coating layer may be crosslinked by crosslinking groups obtained by reacting the reactive groups and optionally a further crosslinking agent;
(c) optionally milling, classifying and/or sieving the granulation of the coated particulate filler; and
(d) optionally further crosslinking the granulation of the coated particulate filler; for providing composite filler particles having a median particle size (D50) of from 1 to 70 µm, wherein reactive groups are transformed into crosslinking groups obtained by reacting reactive groups and optionally a further crosslinking agent, and wherein the particulate filler is the main component by volume of the composite filler particles as further described in EP-A 2 604 247.

The dental composition of the present invention may preferably comprise 0.1 to 85 percent by weight based on the total weight of the composition of particulate filler.

Suitable solvents may be selected from water, alcohols such as methanol, ethanol, propanol (n-, i-), butanol (n-, iso-, ter.-), ketones such as acetone or the like.

The dental composition of the present invention may preferably comprise 5 to 75 percent by weight based on the total weight of the composition of a solvent.

The dental compositions of the present invention may further contain stabilizers, pigments, free radical scavengers, polymerization inhibitors, reactive and nonreactive diluents, coupling agents to enhance reactivity of fillers, rheology modifiers, and surfactants.

Suitable stabilizers may be selected from reducing agents such as vitamin C, inorganic sulfides and polysulfides and the like.

The dental composition according to the present invention may be a one-component composition.

### Example 1

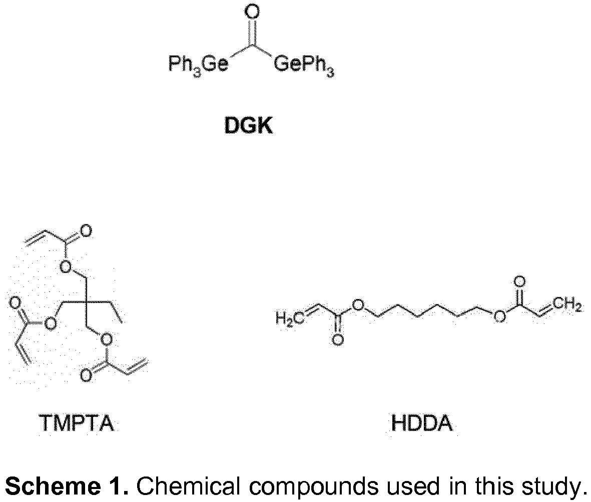

### Preparation of the di(germy)ketone (DGK)

The procedure presented in [Macromol. Rapid Commun. 2010, 31, 473-478] for the synthesis of **DGK** (Scheme 1) was used. To a solution of triphenylgermanium hydride (1 g, 3.28 mmol) in dry THF (3 mL) at -23 °C was added n-BuLi (2.5 M in hexanes, 1.31 mL, 3.28 mmol). After 15 min. at -23 °C, ethyl formate (freshly distilled over CaH₂, 0.58 mL, 1.64 mmol) was added dropwise and the reaction mixture was allowed to warm at room temperature over 15 min. before being quenched with aqueous hydrochloric acid (1M). The organic phase was separated and the aqueous phase was extracted three times with diethyl ether. The combined organic phases were dried over sodium sulfate, filtered and concentrated to give a white powder. ¹H NMR analysis of the crude reaction mixture indicated a 3:7 ratio of bis(triphenylgermyl)methanol /formic acid bis(triphenylgermyl)methyl ester. The latter was dissolved in dry THF (13 mL) and cooled to 0 °C. LiAlH₄ (0.17 g, 4.37 mmol) was then added in one portion and the stirring continued for 1h at 0 °C before being quenched with aqueous hydrochloric acid (1M). The organic phase was separated and the aqueous phase was extracted three times with diethyl ether. The combined organic phases were dried over sodium sulfate, filtered and concentrated to give a colorless oil. The latter was crystallized in a 9:1 EtOH/benzene mixture to give 0.63 g (60%) of bis(triphenylgermyl)methanol. ¹H NMR (CDCl₃, 300 MHz) δ 7.38-7.18 (30 H); 5.38 (s, 1H); 1,5 (1H; OH). ¹³C NMR (CDCl₃, 75 MHz) δ 135.5; 134.0; 130.5; 129.0; 128.6; 128.1; 60.3.

To a solution of bis(triphenylgermyl)methanol (0.25 g, 3.9 mmol) in dry diethyl ether (2.3 mL) were added successively dicyclohexylcarbodiimide (0.19 g, 9.2 mmol) and pyridinium trifluoroacetate (0.09 g, 4.7 mmol). The flask was then carefully protected from light and dry DMSO (111 µL, 15.7 mmol) was added dropwise. The reaction mixture turned pink and the stirring continued overnight. The supernatant liquid was then withdrawn via syringe and filtered through a 0.2 µm syringe filter. The filtrate was then evaporated under vacuum in the absence of light to give bis(triphenylgermyl)methanone as a red solid.

### Photopolymerization procedures:

The laminated films (25 µm thick) deposited on a BaF₂ pellet were irradiated with different light sources: **i)** polychromatic light (incident light intensity: l₀ ≈ 60 mW cm⁻²) delivered by a Xenon lamp (Hamamatsu, L8253, 150 W) and filtered to select a visible light irradiation (λ > 400 nm) and **ii)** a laser diode at 473 nm (100 mW/cm²).

The evolution of the double bond content was continuously followed by real time FTIR spectroscopy at about 1640 cm⁻¹ (Nexus 870, Nicolet) as described in [Fouassier, J.P.; Lalevée, J., Photoinitiators for Polymer Synthesis-Scope, Reactivity, and Efficiency. Wiley-VCH Verlag GmbH & Co. KGaA: 2012].

Trimethylolpropane triacrylate (TMPTA) and 1,6-Hexanediol Diacrylate HDDA (Scheme 1) were selected as benchmark monomers.

### Free radical polymerization initiating ability of DGK:

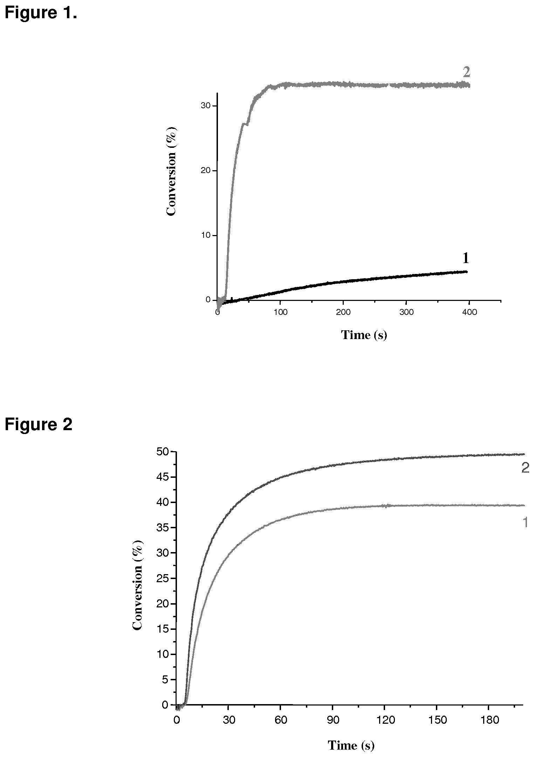

DGK can be an interesting photoinitiator upon visible light exposure i.e. quite good polymerization profiles were obtained for different benchmark acrylate monomers (TMPTA; HDDA) in presence of DGK. No polymerization was observed in absence of DGK clearly showing the role of DGK as photoinitiator for the radical polymerization (Figure 1 curve 1 vs. curve 2). DGK is also very efficient to initiate the polymerization of TMPTA upon a blue light laser diode@ 473 nm (Figure 2). In presence of iodonium salt, the polymerization profile is improved (Figure 2 curve 2 vs. curve 1).

### Example 2: Bis(silyl)ketone as photoinitiator for dental materials.

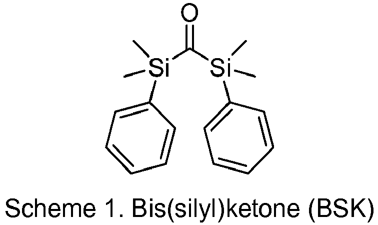

### Preparation of the bis(silvl)ketone (BSK):

The procedure presented in [Kirschning, A.; Luiken, S.; Migliorini, A.; Loreto, M. A.; Vogt, M. Synlett 2009, 3, 429-432] for the synthesis of BSK was adapted. The optimized synthetic route is depicted in the following Scheme 2. The yields for each steps is given in this scheme.

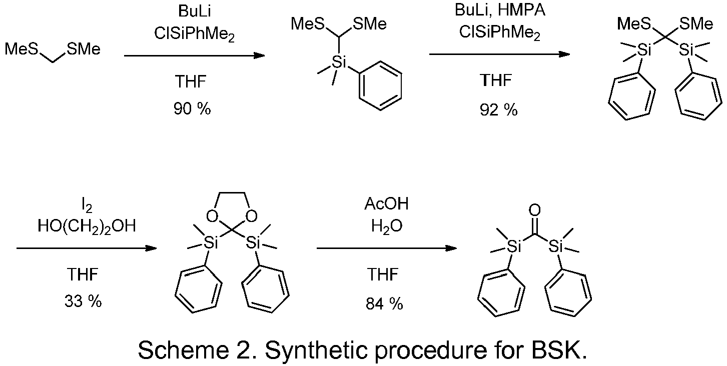

### Irradiation Sources:

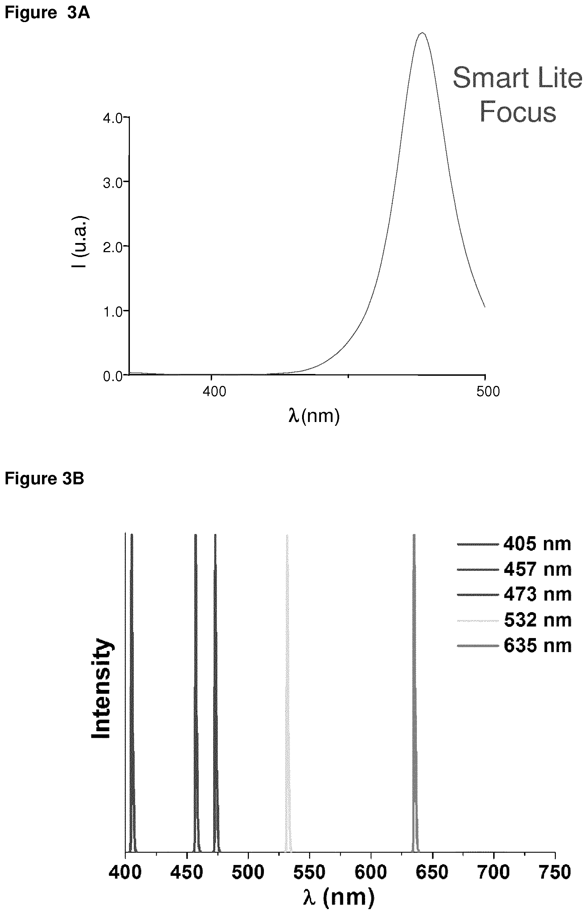

Different lights were used for the irradiation of photocurable samples: blue LED centered at 477 nm (SmartLite Focus - DENTSPLY - ∼ 80 mW cm⁻²) or a laser diode centered at 532 nm (∼100 mW cm⁻²). The emission spectra of the irradiation sources are given in Figure 3.

### ESR Spin Trapping (ESR-ST) Experiments:

ESR-ST experiments were carried out using a Bruker EMX-plus spectrometer (X-band). The radicals were generated at room temperature upon the blue LED exposure (SmartLite Focus from Dentsply) under N₂ and trapped by phenyl-N-tert-butylnitrone (PBN) according to a procedure described in detail in [Fouassier, J.P.; Lalevée, J., Photoinitiators for Polymer Synthesis-Scope, Reactivity, and Efficiency. Wiley-VCH Verlag GmbH & Co. KGaA: 2012]. The ESR spectra simulations were carried out using the WINSIM software.

### Photopolymerization Experiments:

For photopolymerization experiments, the conditions are given in the figure captions. The photocurable formulations were deposited on a BaF₂ pellet in laminate (the formulation is sandwiched between two polypropylene films) (thickness of 25 µm for the formulation) for irradiation with different lights. The evolution of the methacrylate double bond content of the blend Bis-GMA/TEGDMA was continuously followed by the real time FTIR spectroscopy (JASCO FTIR 4100) at about 1640 cm⁻¹ [Tehfe, M. A.; Lalevée, J.; Telitel, S.; Sun, J. F.; Zhao, J. Z.; Graff, B.; Morlet-Savary, F.; Fouassier, J. P. Polymer 2012, 53, 2803-2808. Tehfe, M. A.; Lalevée, J.; Morlet-Savary, F.; Graff, B.; Blanchard, N.; Fouassier, J. P. Macromolecules 2012, 45, 1746-1752].

### Results:

### a) Type I photoinitiator behavior of BSK:

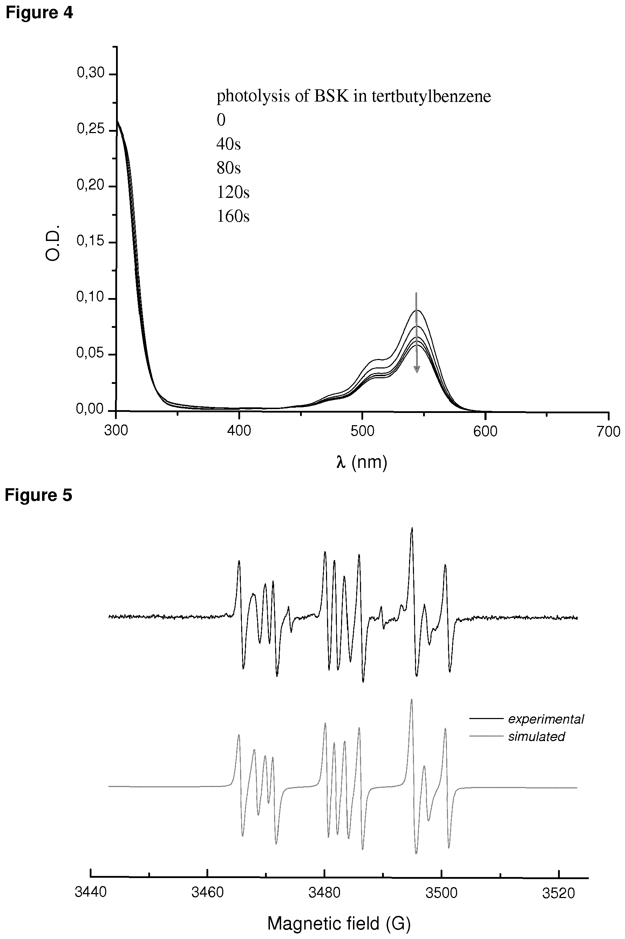

BSK is characterized by good light absorption properties in the 300-600 nm range and can be considered as potential photoinitiator in this spectral range. The maximum of the absorption is found at ∼ 530 nm in acetonitile (Figure 4).

Upon a SmartLite focus exposure, a bleaching of the BSK solution is found (Figure 5). This shows the photosensitivity of this compound upon visible light irradiation.

Electron spin resonance (spin trapping mode) experiments were carried out to investigate the chemical mechanisms for BSK upon visible light exposure. Interestingly, two radicals are generated by light irradiation (Figure 5). The hyperfine coupling constants of these radicals can be ascribed to the silyl radical (a_{N} = 14.7 G; a_{H} = 5.8 G) and acyl radical (a_{N} = 13.6 G; a_{H} = 1.8G) [Fouassier, J.P.; Lalevée, J., Photoinitiators for Polymer Synthesis-Scope, Reactivity, and Efficiency. Wiley-VCH Verlag GmbH & Co. KGaA: 2012] generated by C-Si cleavage process. These latter results unambiguously show the Type I photoinitiator behavior of BSK i.e. free radicals can be generated by homolytic photocleavage process of this derivative (Scheme 3).

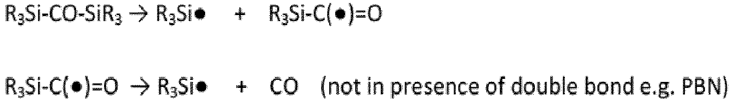

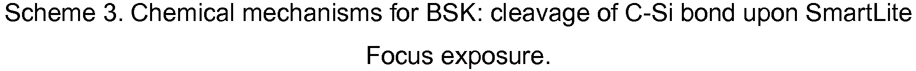

### b) BSK in presence of additive (e.g. DPI):

BSK can also be used in presence of additives (amines, iodonium salts). For example, in presence of DPI, a much faster photolysis of BSK is found than in absence of DPI; this shows the strong BSK/DPI interaction upon a SmartLite Focus irradiation. In ESR spin trapping experiments, the formation of aryl radicals (characterized by a_{N} = 14.2 G; a_{H} = 2.2 G) is clearly observed. A sensitization of DPI decomposition by BSK can be expected according to Scheme 4.

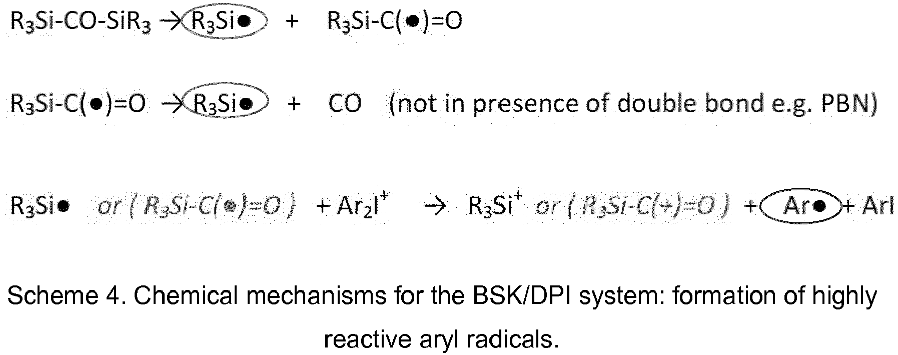

Remarkably, a very good bleaching of the BSK/DPI solution is found; this is useful for the synthesis of colorless polymers.

### c) Free radical polymerization initiating ability of BSK:

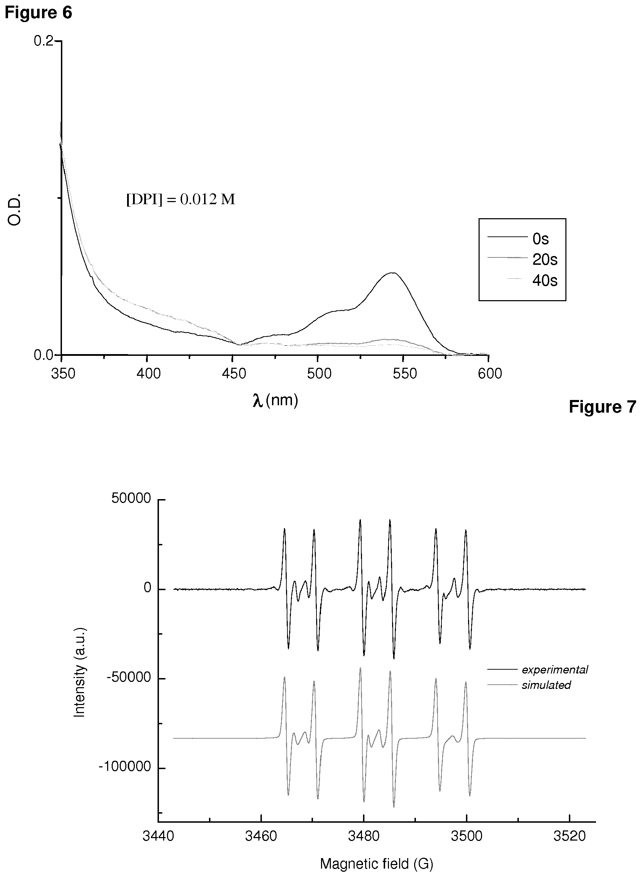

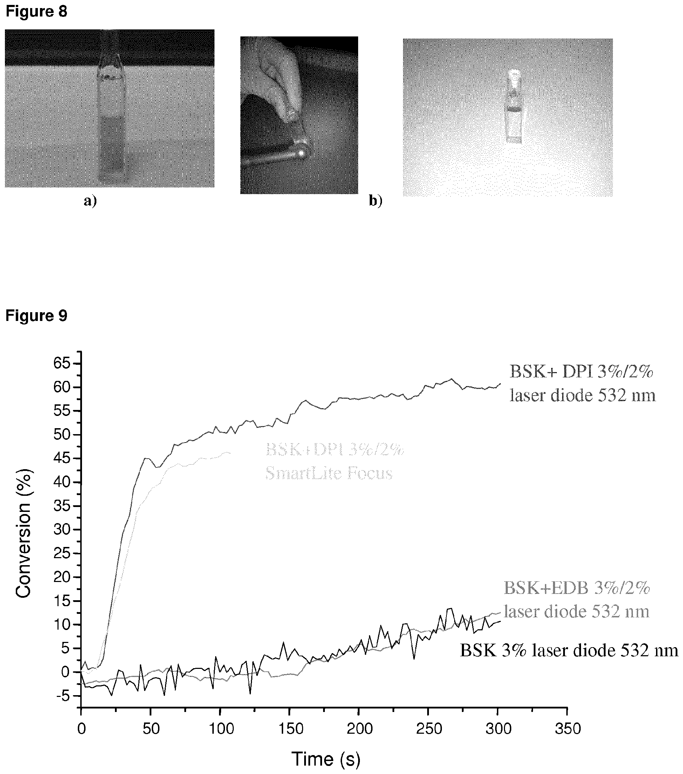

BSK can be an interesting photoinitiator upon visible light. No polymerization was observed in absence of BSK. In presence of BSK, a weak polymerization with a final conversion of -10 % is found after 300 s of irradiation. This polymerization shows the role of BSK as photoinitiator for the radical polymerization. In agreement with the much faster photolysis of BSK in presence of DPI (see Figure 4); the BSK/DPI combination is found as a very efficient initiating system upon visible light (both blue light and green light; Figure 6). The role of amine as additive remains weak for the concentration used here (Figure 8). Using N-vinylcarbazole a relatively low additive effect is also obtained.

### Example 3 - AG 19-56-2

5.0000 g (9.7656 mmol) 2,2-bis[4-[2-hydroxy-3-methacryloyloxypropoxy)phenyl]propane (Bis-GMA), 1.1983 g (4.1853 mmol) triethylene glycol dimethacrylate (TGDMA), 0.1774 g (0.2790 mmol) Bis(triphenylgermyl)keton (TPG) and 0.0047 g (0.0212 mmol) 2,6-di-tert-butyl-p-cresol were mixed homogeneously. Polymerization enthalpy measured with the DSC 7 (Perkin Elmer) is summarized in Table 1.

### Comparative Example 1 - AG 19-27-1

5.0000 g (9.7656 mmol) 2,2-bis[4-[2-hydroxy-3-methacryloyloxypropoxy)phenyl]propane (Bis-GMA), 1.1983 g (4.1853 mmol) triethylene glycol dimethacrylate (TGDMA), 0.0232 g (0.1395 mmol) camphorquinone (CQ), 0.0100 g (0.0517 mmol) 4-(dimethylamino) benzoic acid ethylester (DMABE) and 0.0058 g (0.0216 mmol) 2,6-di-tert-butyl-p-cresol were mixed homogeneously. Polymerization enthalpy measured with the DSC 7 (Perkin Elmer) is summarized in Table 1.

**Table 1: Polymerization enthalpy of a Bis-GMA/TGDMA (70/30 w/w) composition.**

| | | Δ_{R}H [kJ/mol] |
|---|---|---|
| AG 19-56-2 | Example 3 | -46.5 ± 1.6 |
| AG 19-27-1 | Comparative Example 1 | -42.8 ± 2.1 |

The DSC measurements show a 22 % higher polymerization enthalpy of the in germanium hydride basing initiator (Example 1) compared to the CQ/amine system (Comparative Example 1).

## Claims

1. Photocurable dental composition comprising
(a) one or more radical-polymerizable monomers,
(b) 0.1 to 7.0 percent by weight based on the total weight of radical polymerizable monomers in the composition of a compound of the following formula (I):
R¹R²R³X(CO)X'R⁴R⁵R⁶ (I)
wherein
X and X' which may be the same or different, independently represent Si or Ge,
R¹, R², R³, R⁴, R⁵, and R⁶, which may be the same or different, independently represent a hydrocarbon group having 1 to 20 carbon atoms, whereby any two hydrocarbon groups of R¹, R², R³, R⁴, R⁵, and R⁶ may be bonded to each other and form together with either X, X' or the moiety X(CO)X' to which they are attached a 3 to 12- membered heterocyclic ring,
whereby any of the hydrocarbon group or the heterocyclic ring may
- contain one or more keto groups other than the keto group of the X(CO)X' moiety, or
- be substituted by one or more groups selected from halogen atoms, a cyano group, an amino group, or a hydroxy group, and
- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group, and
(c) a particulate filler and/or solvent,
which contains an iodonium salt as an additional initiator.

2. The photocurable dental composition according to claim 1, which comprises less than 2.5 percent by weight based on the total weight of radical polymerizable monomers in the composition of a compound of formula (I).

3. The photocurable dental composition according to claim 1 or 2, wherein the compound of formula (I) has an absorption maximum in the range of from 400 nm to 900 nm.

4. The photocurable dental composition according to any one of the preceding claims, wherein the hydrocarbon group is an aromatic hydrocarbon group selected from a phenyl group and a naphthyl group, which groups may optionally be substituted by one to three groups selected from halogen atoms, a cyano group, an amino group, a hydroxy group, C₁₋₆ alkyl groups and C₁₋₆ alkoxy groups, or wherein the hydrocarbon group is a non-aromatic hydrocarbon group selected from a straight chain or branched alkyl group, a straight chain or branched alkenyl group, or a straight chain or branched alkynyl group.

5. The photocurable dental composition according to any one of the preceding claims, wherein the radical-polymerizable monomers (a) each contain one or two radical-polymerizable groups.

6. The photocurable dental composition according to any one of the preceding claims, which further comprises 5 to 75 percent by weight based on the total weight of the composition of a solvent.

7. The photocurable dental composition according to any one of the preceding claims, which further comprises 0.1 to 85 percent by weight based on the total weight of the composition of particulate filler.

8. The photocurable dental composition according to any one of the preceding claims, which comprises a radical-polymerizable monomer having an acidic group.

9. The photocurable dental composition according to any one of the preceding claims, which is selected from a dental adhesive composition, a dental composite, a resin modified dental cement, and a pit and fissure sealant.

10. The photocurable dental composition according to any one of the preceding claims, which is a one-component composition.

11. The photocurable dental composition according to any one of the preceding claims wherein the X and X' are the same.

12. Use of a compound of the following formula (I):
R¹R²R³Ge(CO)GeR⁴R⁵R⁶ (I)
wherein
R¹, R², R³, R⁴, R⁵, and R⁶, which may be the same or different, independently represent a hydrocarbon group having 1 to 20 carbon atoms, whereby any two hydrocarbon groups of R¹, R², R³, R⁴, R⁵, and R⁶ may be bonded to each other and form together with either Ge or with the moiety Ge(CO)Ge to which they are attached a 3 to 12- membered heterocyclic ring,
whereby the hydrocarbon group or the heterocyclic ring may
- contain one or more keto groups other than the keto group of the moiety Ge(CO)Ge, or
- be substituted by one or more groups selected from halogen atoms, a cyano group, an amino group or a hydroxy group, and
- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group,
for the preparation of a photocurable dental composition as defined in any of claims 1 to 11.

## Patentansprüche

1. Fotohärtbare dentale Zusammensetzung, umfassend
(a) ein oder mehrere radikalisch polymerisierbare Monomere,
(b) 0,1 bis 7,0 Gewichtsprozent, bezogen auf das Gesamtgewicht der radikalisch polymerisierbaren Monomere in der Zusammensetzung, einer Verbindung der folgenden Formel (I):
R¹R²R³X(CO)X'R⁴R⁵R⁶ (I)
wobei
X und X', die gleich oder verschieden sein können, Si oder Ge unabhängig voneinander repräsentieren,
R¹, R², R³, R⁴, R⁵ und R⁶, die gleich oder verschieden sein können, eine Kohlenwasserstoffgruppe mit 1 bis 20 Kohlenstoffatomen unabhängig voneinander repräsentieren, wobei zwei beliebige Kohlenwasserstoffgruppen von R¹, R², R³, R⁴, R⁵ und R⁶ aneinander gebunden sein können und zusammen mit entweder X, X' oder dem X(CO)X'-Rest, an den sie gebunden sind, einen 3- bis 12-gliedrigen heterocyclischen Ring bilden können,
wobei jede der Kohlenwasserstoffgruppen oder der heterocyclische Ring
- eine oder mehrere Ketogruppen anders als die Ketogruppe des X(CO)X'-Rests enthalten kann oder
- durch eine oder mehrere Gruppen substituiert sein kann, die aus Halogenatomen, einer Cyanogruppe, einer Aminogruppe oder einer Hydroxygruppe ausgewählt sind, und
- falls die Kohlenwasserstoffgruppe oder der heterocyclische Ring eine Alkylenkette oder eine Alkenylenkette enthält, ein oder mehrere Kohlenstoffatome in der Alkylenkette oder Alkenylenkette durch ein Sauerstoffatom, ein Schwefelatom, eine Amidgruppe, eine Estergruppe, eine Urethangruppe oder eine NH-Gruppe und
(c) einen teilchenförmigen Füllstoff und/oder ein teilchenförmiges Lösungsmittel,
der/das ein lodoniumsalz als zusätzlicher Initiator enthält, substituiert sein können.

2. Fotohärtbare dentale Zusammensetzung nach Anspruch 1, die weniger als 2,5 Gewichtsprozent, bezogen auf das Gesamtgewicht der radikalisch polymerisierbaren Monomere in der Zusammensetzung, einer Verbindung der Formel (I) umfasst.

3. Fotohärtbare dentale Zusammensetzung nach Anspruch 1 oder 2, wobei die Verbindung der Formel (I) ein Absorptionsmaximum im Bereich von 400 nm bis 900 nm aufweist.

4. Fotohärtbare dentale Zusammensetzung nach einem der vorhergehenden Ansprüche, wobei die Kohlenwasserstoffgruppe eine aromatische Kohlenwasserstoffgruppe ist, die aus einer Phenylgruppe und einer Naphthylgruppe ausgewählt ist, wobei diese Gruppen optional durch eine bis drei Gruppen substituiert sein können, die aus Halogenatomen, einer Cyanogruppe, einer Aminogruppe, einer Hydroxygruppe, C₁₋₆-Alkylgruppen und C₁₋₆-Alkoxygruppen ausgewählt sind, oder wobei die Kohlenwasserstoffgruppe eine nichtaromatische Kohlenwasserstoffgruppe ist, die aus einer geradekettigen oder verzweigten Alkylgruppe, einer geradekettigen oder verzweigten Alkenylgruppe oder einer geradkettigen oder verzweigten Alkinylgruppe ausgewählt ist.

5. Fotohärtbare dentale Zusammensetzung nach einem der vorhergehenden Ansprüche, wobei die radikalisch polymerisierbaren Monomere (a) jeweils eine oder zwei radikalisch polymerisierbare Gruppen enthalten.

6. Fotohärtbare dentale Zusammensetzung nach einem der vorhergehenden Ansprüche, die ferner 5 bis 75 Gewichtsprozent, bezogen auf das Gesamtgewicht der Zusammensetzung, eines Lösungsmittels umfasst.

7. Fotohärtbare dentale Zusammensetzung nach einem der vorhergehenden Ansprüche, die ferner 0,1 bis 85 Gewichtsprozent, bezogen auf das Gesamtgewicht der Zusammensetzung, eines teilchenförmigen Füllstoffs umfasst.

8. Fotohärtbare dentale Zusammensetzung nach einem der vorhergehenden Ansprüche, die ein radikalisch polymerisierbares Monomer mit einer sauren Gruppe umfasst.

9. Fotohärtbare dentale Zusammensetzung nach einem der vorhergehenden Ansprüche, die aus einer Zahnklebstoffzusammensetzung, einem dentalen Kompositmaterial, einem harzmodifizierten Dentalzement und einem Gruben- und Fissurenversiegelungsmittel ausgewählt ist.

10. Fotohärtbare dentale Zusammensetzung nach einem der vorhergehenden Ansprüche, die eine einkomponentige Zusammensetzung ist.

11. Fotohärtbare dentale Zusammensetzung nach einem der vorhergehenden Ansprüche, wobei X und X' gleich sind.

12. Verwendung einer Verbindung der folgenden Formel (I):
R¹R²R³Ge(CO)GeR⁴R⁵R⁶ (I)
wobei
R¹, R², R³, R⁴, R⁵ und R⁶, die gleich oder verschieden sein können, eine Kohlenwasserstoffgruppe mit 1 bis 20 Kohlenstoffatomen unabhängig voneinander repräsentieren, wobei zwei beliebige Kohlenwasserstoffgruppen von R¹, R², R³, R⁴, R⁵ und R⁶ aneinander gebunden sein können und zusammen mit entweder Ge oder dem Ge(CO)Ge-Rest, an den sie gebunden sind, einen 3- bis 12-gliedrigen heterocyclischen Ring bilden können,
wobei die Kohlenwasserstoffgruppe oder der heterocyclische Ring
- eine oder mehrere Ketogruppen anders als die Ketogruppe des Ge(CO)Ge-Rests enthalten kann oder
- durch eine oder mehrere Gruppen substituiert sein kann, die aus Halogenatomen, einer Cyanogruppe, einer Aminogruppe oder einer Hydroxygruppe ausgewählt sind, und
- falls die Kohlenwasserstoffgruppe oder der heterocyclische Ring eine Alkylenkette oder eine Alkenylenkette enthält, ein oder mehrere Kohlenstoffatome in der Alkylenkette oder Alkenylenkette durch ein Sauerstoffatom, ein Schwefelatom, eine Amidgruppe, eine Estergruppe, eine Urethangruppe oder eine NH-Gruppe
zur Herstellung einer fotohärtbaren dentalen Zusammensetzung gemäß einem der Ansprüche 1 bis 11 substituiert sein können.

## Revendications

1. Composition dentaire photodurcissable comprenant
(a) au moins un monomère polymérisable par un procédé radicalaire,
(b) 0,1 à 7,0 pour cent en poids, sur la base du poids total des monomères polymérisables par un procédé radicalaire de la composition, d'un composé de formule (I) suivante :
R¹R²R³X(CO)X'R⁴R⁵R⁶ (I)
dans laquelle
X et X' qui peuvent être identiques ou différents, représentent indépendamment Si ou Ge,
R¹, R², R³, R⁴, R⁵ et R⁶, qui peuvent être identiques ou différents, représentent indépendamment un groupe hydrocarboné comptant 1 à 20 atomes de carbone, moyennant quoi deux groupes hydrocarbonés quelconques de R¹, R², R³, R⁴, R⁵ et R⁶ peuvent être liés l'un à l'autre et former ensemble avec X, X' ou avec la fraction X(CO)X' à laquelle ils sont liés, un cycle hétérocyclique à 3 à 12 chaînons,
moyennant quoi l'un quelconque du groupe hydrocarboné ou du cycle hétérocyclique peut
- contenir au moins un groupe céto autre que le groupe céto du fragment X(CO)X' ou
- être substitué par au moins un groupe choisi parmi les atomes d'halogène, un groupe cyano, un groupe amino ou un groupe hydroxy et
- dans le cas où le groupe hydrocarboné ou le cycle hétérocyclique contient une chaîne alkylène ou une chaîne alcénylène, au moins un atome de carbone de la chaîne alkylène ou de la chaîne alcénylène peut être remplacé par un atome d'oxygène, un atome de soufre, un groupe amide, un groupe ester, un groupe uréthane ou un groupe NH et
(c) une charge particulaire et/ou un solvant,
qui contient un sel d'iodonium en tant qu'initiateur supplémentaire.

2. Composition dentaire photodurcissable selon la revendication 1, qui comprend moins de 2,5 pour cent en poids sur la base du poids total de monomères polymérisables par un procédé radicalaire dans la composition d'un composé de formule (I).

3. Composition dentaire photodurcissable selon la revendication 1 ou 2, dans laquelle le composé de formule (I) présente un maximum d'absorption dans la plage de 400 nm à 900 nm.

4. Composition dentaire photodurcissable selon l'une quelconque des revendications précédentes, dans laquelle le groupe hydrocarboné est un groupe hydrocarboné aromatique choisi parmi un groupe phényle et un groupe naphtyle, lesquels groupes peuvent éventuellement être substitués par un à trois groupes choisis parmi des atomes d'halogène, un groupe cyano, un groupe amino, un groupe hydroxy, des groupes alkyle en C₁₋₆ et des groupes alcoxy en C₁₋₆ ou dans laquelle le groupe hydrocarboné est un groupe hydrocarboné non aromatique choisi parmi un groupe alkyle à chaîne linéaire ou ramifié, un groupe alcényle à chaîne linéaire ou ramifié ou un groupe alcynyle à chaîne linéaire ou ramifié.

5. Composition dentaire photodurcissable selon l'une quelconque des revendications précédentes, dans laquelle les monomères polymérisables par un procédé radicalaire (a) contiennent chacun un ou deux groupes polymérisables par un procédé radicalaire.

6. Composition dentaire photodurcissable selon l'une quelconque des revendications précédentes, qui comprend en outre 5 à 75 pour cent en poids sur la base du poids total de la composition d'un solvant.

7. Composition dentaire photodurcissable selon l'une quelconque des revendications précédentes, qui comprend en outre 0,1 à 85 pour cent en poids sur la base du poids total de la composition de charge particulaire.

8. Composition dentaire photodurcissable selon l'une quelconque des revendications précédentes, qui comprend un monomère polymérisable par un procédé radicalaire comportant un groupe acide.

9. Composition dentaire photodurcissable selon l'une quelconque des revendications précédentes, qui est choisie parmi une composition adhésive dentaire, un composite dentaire, un ciment dentaire modifié à la résine et un scellant pour puits et pour fissures.

10. Composition dentaire photodurcissable selon l'une quelconque des revendications précédentes, qui est une composition à un constituant.

11. Composition dentaire photodurcissable selon l'une quelconque des revendications précédentes, dans laquelle X et X' sont identiques.

12. Utilisation d'un composé de la formule générale (I) :
R¹R²R³Ge(CO)GeR⁴R⁵R⁶ (I)
dans laquelle
R¹, R², R³, R⁴, R⁵ et R⁶, qui peuvent être identiques ou différents, représentent indépendamment un groupe hydrocarboné comptant 1 à 20 atomes de carbone, moyennant quoi deux groupes hydrocarbonés quelconques de R¹, R², R³, R⁴, R⁵ et R⁶ peuvent être liés l'un à l'autre et former ensemble, avec Ge ou avec le fragment Ge(CO)Ge auquel ils sont attachés, un cycle hétérocyclique à 3 à 12 chaînons,
moyennant quoi le groupe hydrocarboné ou le cycle hétérocyclique peut
- contenir au moins un groupe céto autre que le groupe céto du fragment Ge(CO)Ge ou
- être substitué par au moins un groupe choisi parmi les atomes d'halogène, un groupe cyano, un groupe amino ou un groupe hydroxy et
- dans le cas où le groupe hydrocarboné ou le cycle hétérocyclique contient une chaîne alkylène ou une chaîne alcénylène, au moins un atome de carbone de la chaîne alkylène ou de la chaîne alcénylène peut être remplacé par un atome d'oxygène, un atome de soufre, un groupe amide, un groupe ester, un groupe uréthane ou un groupe NH,
pour la préparation d'une composition dentaire photodurcissable telle que définie dans l'une quelconque des revendications 1 à 11.
